# EUROPEAN PATENT APPLICATION

(11) **EP 3 001 422 A1**
(43) Date of publication of application: **30.03.2016**
(21) Application number: 15186424.6
(22) Date of filing: 23.09.2015
(51) Int. Cl.: G11B 27/34, G06F 3/01

(54) **MEDIA PLAYER AUTOMATED CONTROL BASED ON DETECTED PHYSIOLOGICAL PARAMETERS OF A USER**

(30) Priority: 25.09.2014 US 201414496976
(71) Applicant: Harman International Industries, Inc., Stamford, CT 06901 (US)
(72) Inventor: CHATTERJEE, Dibyendu, Stamford, CT Connecticut 06901 (US); INDUKUMAR, Sowrabha Mysore, Stamford, CT Connecticut 06901 (US); CHAKRABORTY, Sumit, Stamford, CT Connecticut 06901 (US)
(74) Representative: Bertsch, Florian Oliver

(57) **Abstract**

System, controller, and computer readable medium for controlling a presentation of audio and video information to a user. Physiological parameters of the user can be monitored in real time to determine changes in activity. A video presentation and/or an audio presentation can be modified based on the determined change in activity. Additionally, a user interface can be modified based on the determined change in activity.

## Description

### BACKGROUND

The present invention relates to digital media players, and more specifically, to controlling at least one of an audio presentation, a video presentation, and a user interface, based on detected physiological parameters of a user.

### SUMMARY

According to various embodiments, a system can include a computer processor and at least one physiological sensor in communication with the computer processor. The at least one physiological sensor can detect at least one physiological parameter of a user. The system can also include at least one of a video display that can display video images and an audio transducer that can generate audio signals. The system can also include a user interface in communication with the computer processor. The user interface includes at least one control that enables the user to control one or more aspects of at least one of video images displayed on the video display and audio signals output by the audio transducer. The computer processor can monitor the detected physiological parameters for changes in a detected physiological parameter or physiological state of the user. Upon detecting such a change in physiological state or a physiological parameter, the computer processor can modify a presentation aspect of at least one of the displayed video image, the audio signal, and/or the user interface.

According to various embodiments, a controller can include a first signal input that receives a first physiological signal that indicates a physiological state of a user. The controller can also include a second signal input configured to receive a user control signal that indicates a command related to at least one of a display of a video image and an output of an audio signal. The controller can also include a third signal input configured to receive at least one of a video image signal and an audio signal. The controller can also include a first signal output configured to output a modification control signal. The controller can also include computer logic that is programmed to provide to the first signal output the at least one of the video image signal and the audio signal. The computer logic is also programmed to issue the modification control signal from the first output to modify at least one presentation aspect of the at least one of the video image signal and the audio signal upon the first signal input indicating a change in the physiological state of the user.

According to various embodiments, a computer readable medium can include a program that can be executed on one or more processors. When executed, the program can perform an operation that includes outputting at least one of a video signal and an audio signal. The operation can also include receiving at least one indication of a physiological state of a user. The operation can also include modifying the output of at least one of the video signal and audio signal upon receiving at least one indication of a change to the physiological state of the user.

According to various embodiments, a system can include a computer processor and at least one physiological sensor in communication with the computer processor. The at least one physiological sensor can detect at least one physiological parameter of a user. The system can also include an audio transducer that can generate audio signals. The computer processor can monitor the detected physiological parameters for changes in a detected physiological parameter or physiological state of the user. Upon detecting such a change in physiological state or a physiological parameter, the computer processor can modify a presentation aspect of the audio signal.

### BRIEF DESCRIPTION OF THE SEVERAL VIEWS OF THE DRAWINGS

Figure 1 illustrates an exemplary scenario in which a docking station according to various embodiments is arranged in a room with a user;
Figure 2 illustrates an embodiment of a docking station controller in a smart phone;
Figure 3 illustrates a block diagram of an embodiment of a controller;
Figure 4 illustrates a flow chart of a process that embodiments of a controller can implement;
Figures 5A and 5B illustrate an embodiment of a modified user interface based on detected physiological changes of a user;
Figures 6A and 6B illustrate another embodiment of a modified user interface based on detected physiological changes of a user; and
Figure 7 illustrates a display of an event log that was created while an embodiment of a controller determined that a user was not to be disturbed, based on data from physiological sensors.

### DETAILED DESCRIPTION

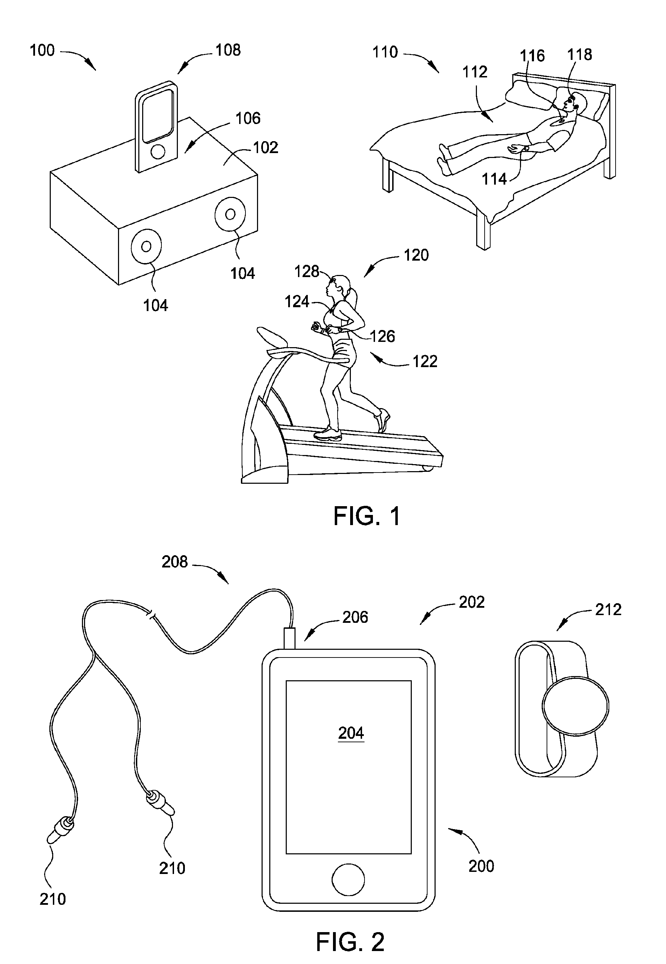

Figure 1 illustrates an embodiment of a docking station 102 that can be used in combination with a media player 108 (e.g., an iPod or an iPhone). The docking station 102 can include a docking port 106 that can physically connect to the media player 108. Alternatively, the docking port 106 can be a wireless connection to the media player 108, such as a Bluetooth wireless connection. The docking port 106 can include control protocols that enable the docking station 102 to control aspects of the media player 108. For example, the docking port 106 may incorporate iAP and iAP2 protocols developed by Apple® to control an iPod® or iPhone® docked to the docking station 102. The control protocols can allow the docking station 102 to control the selection of music tracks, pause music, play music, fast forward, reverse, or the like without manipulating physical controls (e.g., buttons, scroll wheels, and touch pads) on the media player 108.

The docking station 102 can include audio transducers 104 that can output an audio signal (e.g., from the media player 108). The docking station 102 can also include a video display that can display a video image (e.g., from the media player 108). The docking station 102 can be arranged in a room or other space 100 in which a user is engaged in activity. For example, a user 112 may be lying in a bed 110. The user 112 can be wearing one or more physiological sensors that can detect physiological states of the user 112. For example, the user 112 may be wearing a wrist strap 114 that includes a heart rate monitor and/or a thermometer. As another example, the user 112 may be wearing a chest strap 116 or other wearable sensor that detects heart rate and/or body temperature. As another example, the user 112 may be wearing a headband 118 that incorporates a body temperature sensor and/or an electroencephalography (EEG) sensor. The docking station 102 can be in wireless communication with one or more of the sensors 114, 116, and 118 to monitor one or more physiological states of the user 112.

In the event that the docking station monitors a change in a physiological state of the user 112 (e.g., from one or more of the sensors 114, 116, and 118), the docking station 102 can modify operation of the media player 108. For example, a heart rate sensor worn by the user 112 may detect a slowing heart beat and/or a body temperature sensor may detect a reduced body temperature, which may indicate that the user 112 has fallen asleep. As a result, the docking station 102 may automatically adjust a presentation aspect of the media player 108. For example, a presentation aspect of an audio signal could be the volume of the audio signal, and the docking station 102 could decrease the volume of music being played from the media player 108 through the audio transducers 104. As another example, a presentation aspect of the audio signal could be a state of the audio signal, such as "play," "stop," "pause," "mute," *etc.,* and the docking station 102 may automatically stop or pause an audio signal from the media player 108 that is being played through the audio transducers 104. In instances where the media player 108 is a smart phone (e.g., an iPhone), the docking station 102 could also mute any ring tones associated with phone calls, text messages, or the like while the user 112 is asleep.

In various instances, the user 112 may use the docking station 102 or a smart phone in communication with the docking station 102 as an alarm to wake the user from sleep. For example, the user may set the docking station to play a song to wake the user. In various embodiments, the physiological sensors (e.g., sensors 114, 116, and 118 discussed above) can detect a sleep state of the user. For example, the physiological sensors may detect that the user is sleeping very lightly or is in a rapid eye movement (REM) sleep cycle when he is scheduled to be awakened. If the user is sleeping lightly, then the docking station 102 may play the song normally because any disturbance should be sufficient to wake the user. However, if the user is in a REM sleep cycle, then the song may be insufficient to wake the user from such a deep sleep. In such instances in which the user is detected to be in REM sleep, the docking station 102 can modify the song so that it is more likely to wake the user. For example, the docking station 102 may modify or adjust equalizer settings to emphasize frequencies in the song that may be particularly disruptive to the user, thereby causing the user to wake from REM sleep.

In various embodiments, if the docking station 102 detects that a user 112 has fallen asleep, then the system may also record a video or audio that the user 112 is watching or listening to at the time of falling asleep. For example, the docking station may be able to control operation of a television, cable box, *etc.* through an infrared transmitter, radio transmitter or the like. If the user 112 falls asleep while watching a program on the television, the smart phone may send control commands to turn off the television and to record the program on a DVR attached to the television. The docking station 102 may also note the time that the user fell asleep and provide that time to the user 112 when he wakes up so the user may know when to start watching the program to pick up where he fell asleep. The docking station 102 could also use a recognition program, such as Shazam®, to recognize a program being watched or listened to by the user to identify the program and a time within the program that the user fell asleep. For example, the system could determine that the user 112 fell asleep twelve minutes into a rerun of a particular television show.

Figure 1 also illustrates a user 122 on a treadmill 120. Again, the user 122 may be wearing one or more sensors 124, 126, and 128 that enable monitoring one or more physiological states of the user 112. For example, the user 122 may be wearing a wrist strap 124 that includes a heart rate monitor and/or a thermometer. As another example, the user 122 may be wearing a chest strap 126 or other wearable sensor that detects heart rate and/or body temperature. As another example, the user 122 may be wearing a headband 128 that incorporates a body temperature sensor and/or an electroencephalography (EEG) sensor. Again, the sensors 124, 126, and 128 can be in wireless communication with the docking station 102. In the event that one or more of the sensors 124, 126, and 128 detect a change in a physiological state of the user 122, the docking station 102 can modify operation of the media player 108. For example, if the user 122 is walking on the treadmill 120, then his heart rate may be slightly elevated. However, if the user 122 begins to run on the treadmill 120, then his heart rate may be significantly elevated. Upon detecting the increased heart rate of the user 122, the docking station 102 could increase the volume of audio being output by the audio transducers 104 to compensate for increased noises associated with running on the treadmill 120, such as louder breathing sounds, louder footfalls, and louder machinery noises from the treadmill 120. The increased noises associated with running can also affect what the user can hear. For example, when the user is running, low-volume portions of songs may not be heard by the user. Similarly, very high and very low frequencies may not be heard by the user. The docking station 102 can compress or reduce the dynamic range of volume and/or frequencies in the song in response to the one or more sensors 124, 126, and 128 detecting that the user is running. For example, the docking station 102 may automatically increase the volume of low-volume portions of songs. As another example, the docking station 102 may modify frequencies of the audio signal (e.g., skew low frequencies in a song to higher frequencies and skew high frequencies in the song to lower frequencies).

Upon detecting the increased heart rate of the user 122, the docking station 102 could also change the audio being played. For example, the user 122 may have arranged a workout playlist on the media player 108. Upon detecting an increased heart rate, the docking station 102 could automatically select and play songs from the workout playlist.

Figure 2 illustrates an embodiment of a system incorporated within a smart phone 200, tablet, or the like. The smart phone 200 can incorporate a computer processor and memory that can store music files, video files, and the like. The memory can also store application files that can be executed by the processor. The smart phone 200 can include a touch screen display 204 that a user can interact with to control aspects of the smart phone 200. For example, the touch screen display 204 may display various icons and/or graphical user interfaces that enable the user to touch regions of the screen to select applications, select audio tracks, adjust audio volume, accept or reject phone calls, etc. Headphones 208 can be connected to the smart phone 200 through a headphone jack 206. The headphones 208 can be ear buds 210, over-the-ear headphones, or on-the-ear headphones, for example. In various embodiments, the headphones 208 can be connected to the smart phone 200 through a wireless connection (e.g., a Bluetooth® connection). One or more physiological sensors 212 can also be connected to the smart phone 200. As described above, the one or more physiological sensors 212 can be arranged in a wrist strap, chest strap, headband, or the like, and may measure heart rate, body temperature, EEG data, or the like for a user.

In various embodiments, a system or aspects of a system can be arranged in headwear, such as Google® Glass®. The headwear can include audio transducers that can extend from the headwear to a user's ears (when the headwear is worn). The headwear can also include a video display screen, which can be arranged in front of the user's eye (e.g., when the headwear is worn) to present video information to the user. The headwear can also incorporate an eye tracking sensor that can track an eye gaze direction of the user, and the user can provide control commands by looking at various regions of the video display screen. For example, if the user is listening to music or watching a video, then the video display screen could include icons for pausing, fast forwarding, *etc.* The user can provide a command by looking at the appropriate icon for a period of time (e.g., and without limitation, one half second). The headwear can include memory that stores various computer data structures, such as MP3 files and MPEG files that play audio and video, respectively, when processed by a processor. The headwear can also include a data transceiver, such as a Wi-Fi transceiver, a cellular data transceiver, and/or a Bluetooth® data transceiver, to download and/or stream audio and/or video from a remote source. For example, the headwear could be in communication with a portable media player carried by the user that has the data structures stored thereon. As another example, the headwear may connect to a remote computer system that stores audio and/or video files (e.g., Pandora.com or Crackle.com) and download and/or stream music and/or videos from the remote computer system.

The headwear can incorporate and/or be in communication with one or more physiological sensors to determine physiological changes in the user. For example, an EEG sensor and a thermometer may be incorporated into the headwear. As another example, a heart rate sensor (e.g., attached to the user's chest or attached to a watch worn by the user) may wirelessly communicate with the headwear (e.g., via a Bluetooth® connection). As discussed above and in additional detail below, the headphones can affect presentation aspects of an audio presentation, a video presentation, and/or a user interface based on detected changes to physiological parameters of the user. For example, the size and/or arrangement of graphical icons used by the user to control the video and/or audio presentation may be modified based on the detected changes to physiological parameters of the user.

In various embodiments, a system or aspects of a system can be incorporated into a remote control used to control a television, stereo, DVD player, or the like. The remote control could include physiological sensors on its surface and/or could be in communication with one or more physiological sensors, such as sensors 124, 126, and 128 discussed above. In the event the remote control detects a change in physiological state of a user, the remote control could automatically adjust presentation aspects of the television, stereo, *etc.* For example, the remote control could automatically turn off the television if it detects that the user has fallen asleep (assuming that the user is holding or touching the remote control with built in physiological sensors or is wearing physiological sensors in communication with the remote control). The remote control could also automatically record the program the user is watching when he falls asleep. As another example, the remote control could automatically increase the volume of the television and/or the stereo if it detects that the user is running on a treadmill.

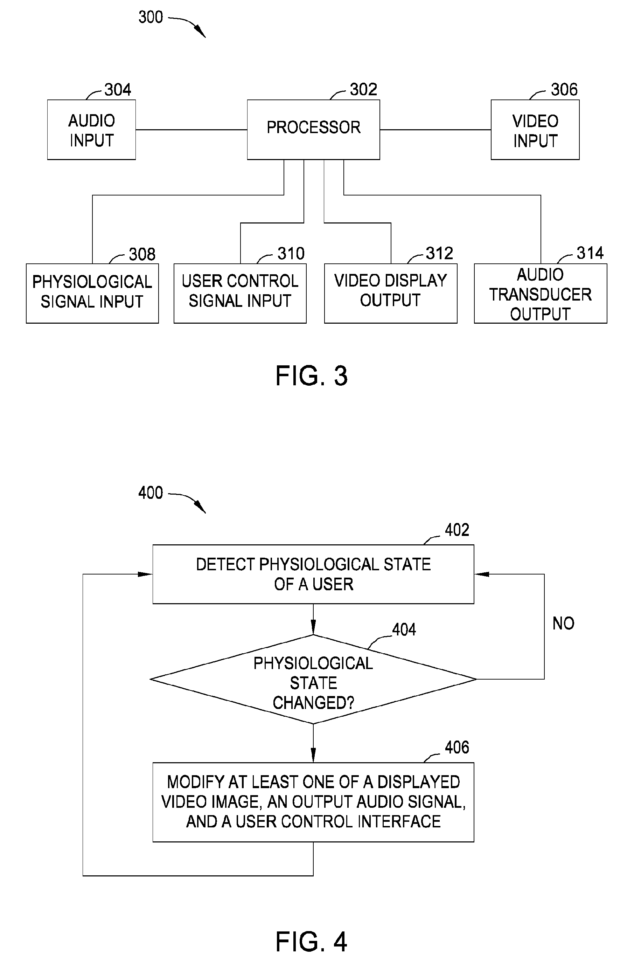

Figure 3 illustrates a block diagram for an embodiment of a controller 300 that can adjust presentation aspects of audio, video, and/or a user interface based on a detected physiological state for a change to a detected physiological state of the user. The controller 300 includes a processor, such as a general computer processor or an application-specific integrated circuit. The processor 302 can be in communication with an audio input 304 and/or a video input 306. For example, with respect to the docking station 102 shown in Figure 1, the audio input 304 and the video input 306 can be the docking port 106 through which the media player 108 connects to the docking station 102. The media player 108 can send to a processor in the docking station 102 audio, such as music or a ring tone (e.g., when a telephone call or a text messages received), or video, such as a movie file, video file, or the like. As another example, with respect to the smart phone 200 shown in Figure 2, the source(s) for the audio input 304 and the video input 306 can be internal to the smart phone 200. For example, audio files and video files may be stored in a computer memory within the smart phone 200, and may be processed by the processor 302 for playback. The controller 300 can also include a physiological signal input 308 that receives sensor signals from the user worn sensors (such as sensors 114, 116, 118, 124, 126, and 128 shown in figure 1 and sensor 212 shown in figure 2). As described above, the sensors can be wirelessly connected to the physiological signal input 308 of the controller 300. For example, the sensors may communicate with the physiological signal input 308 via a Bluetooth connection. As another example, the physiological signal input 308 may include a Wi-Fi connection or a cellular data connection, and the various sensors can also include a Wi-Fi connection or a cellular data connection such that the sensors can communicate with the physiological signal input 308.

The controller 300 can also include a user control signal input 310. For example, the docking station 102 shown in Figure 1 can include one or more buttons, knobs, dials, or the like that the user can manipulate to affect operation of the media player 108. For example, the user may turn a dial to adjust the volume of music being played through the audio transducers 104. As another example, the user may press a button to initiate playback of music, pause music, skip to the next track, repeat a track, or the like. In various instances, the docking station 102 may include a remote control that includes various buttons for controlling at least some of the functions of the docking station 102 and the media player 108 (through the docking station 102). As another example, the smart phone 200 can include a touch screen display 204. As described above, the processor 302 can display on the touch screen display 204 various icons and/or graphical user interfaces, such that a user can touch the screen to manipulate the icons and/or interfaces to affect operation of the smart phone 200. The processor 302 can receive signals from the touch screen display 204 that indicate control instructions (provided by the user touching the display screen 204). The controller 300 can also include a video display output 312 and an audio transducer output 314. In the docking station 102 shown in Figure 1, the audio transducer output 314 can output an audio signal to the audio transducers 104 to generate sound, such as music or ring tones. Additionally, the video display output 312 can output a video image signal to a display screen of the mobile device 108. In the smart phone 200 shown in Figure 2, the audio transducer output 314 can output an audio signal to the headphones 208 connected to the smart phone 200 or to a speaker of the smart phone 200. Additionally, the video display output 312 can output a video image signal to the display screen 204.

The controller 300 can be implemented in a docking station, an audio receiver/amplifier, or the like. The controller 300 can also be implemented in a smart phone. In various embodiments, the processor 302 of the controller 300 can include an application-specific processor dedicated to performing the operations of the controller 300. In various embodiments, the processor 302 can be a general purpose processor. For example, the processor 302 may be a processor used by the docking station to support operation of the docking station, and the controller 300 can be at least partially implemented as software or an application that executes on the docking station processor. Similarly, the processor 302 may be a processor used by a smart phone to support operation of the docking station, and the controller 300 can be at least partially implemented as software or an application that executes on the smart phone processor.

The controller 300 can enable operation of a device, such as the docking station 102 shown in Figure 1 with a smart phone shown in Figure 2, to be modified based on changing physiological states of a user. Figure 4 illustrates a process 400 that the controller 300 can implement to provide such modification. In block 402 of the process 400, the controller 302 can detect a physiological state of a user. For example, a user may be wearing a heart rate monitor that is in communication with the physiological signal input 308 of the controller 300. In block 404, the controller 300 can determine whether there has been a change to the detected physiological state. For example, if the user begins running on a treadmill, then the heart rate monitor may communicate an increased heart rate to the controller 300 through the physiological signal input 308. In the event a change in physiological state is detected, then in block 406, the controller 300 can modify at least one of a displayed video image, and output audio signal, and a user control interface.

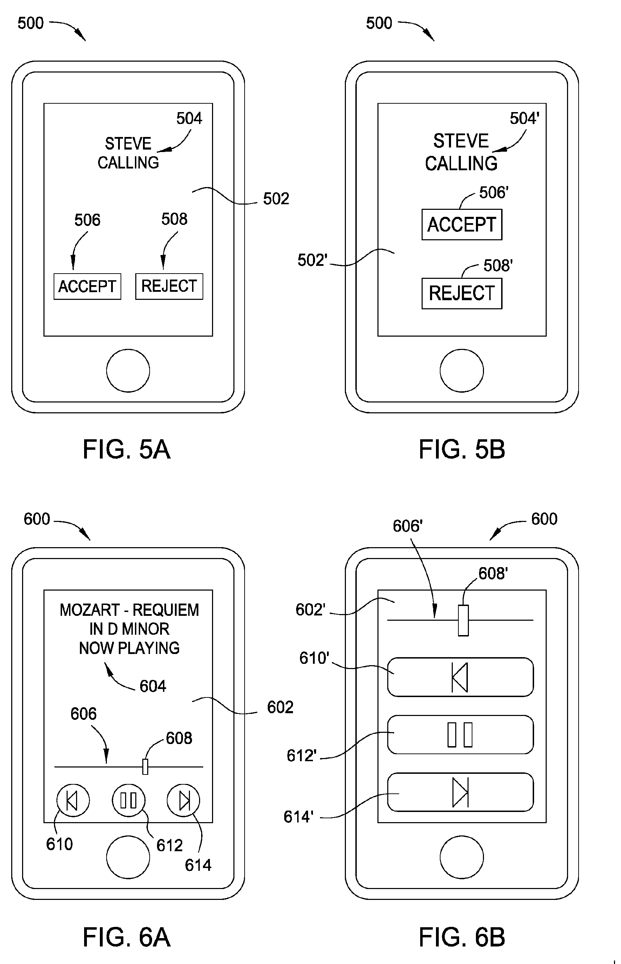

Figures 5A and 5B illustrate an example of how a controller may modify presentation aspects of a user interface. In the scenario depicted in Figures 5A and 5B, a user is receiving a telephone call on a smart phone from Steve. Figure 5A illustrates a smart phone 500 with a touch screen display 502 in a baseline or unmodified mode of operation. As depicted in Figure 5A, the touch screen display 502 includes a first field 504 identifying Steve as calling. The touch screen display 502 also includes a first icon 506 that denotes a first region of the touch screen display 502 that the user can touch to accept the call and a second icon 508 that the notes a second region of the touch screen display 502 that the user can touch to reject the call. In addition, the smart phone 500 may be generating a ring tone through headphones worn by the user and/or an external speaker phone.

In the example in which the user begins running on a treadmill, a controller in the smart phone 500 may detect an increased heart rate of the user, for example. As a result, the controller can modify presentation aspects of the user interface on the touch screen display 502. Figure 5B illustrates the smart phone 500 wherein the user interface on the touch screen display 502' has been modified. For example, the first field 504' that states "STEVE CALLING" has been enlarged (relative to the baseline size) such that the text may be easier to read while the user is running on the treadmill. Similarly, the accept icon 506' and the reject icon 508' have been enlarged (relative to the baseline sizes) so that the user can indicate whether to accept or reject the phone call with a less-precise touch to the touch screen display 502'. In addition, the controller in the smart phone 500 may increase the volume of the generated ring tone (through the headphones and/or speakerphone) to enable the user running on the treadmill can hear the ring tone.

Figures 6A and 6B illustrate another example of how a controller may modify presentation aspects of a user interface. In this scenario depicted in Figures 6A and 6B, a user is listening to music on a portable media player 600, such as an iPod® or an iPhone®. Figure 6A illustrates the media player 600 with a touch screen display 602 in a baseline mode of operation. As depicted in Figure 6A, the touch screen display 602 includes a first field 604 that identifies a song being played. The touch screen display 602 can include a graphical user interface 606, which depicts a slider bar 608 that a user may touch and move to adjust the volume of the generated sound of the song. The touch screen display 602 can also include icons 610, 612, and 614 that denote regions of the touch screen display 602 that a user can touch to restart the song, pause the song, or skip to the next song, respectively. The audio for the song can be generated by headphones worn by the user and/or by a speakerphone of the media player 600.

In the example in which the user begins running on a treadmill, a controller in the media player 600 may detect an increased heart rate of the user, for example. As a result, the controller can modify the user interface on the touch screen display 602. Figure 6B illustrates the media player 600 wherein the user interface on the touch screen display 602 prime has been modified. For example, the first field 604 that identifies the song being played has been omitted from the modified user interface. The graphical user interface 606 and the icons 610', 612', and 614' have been rearranged and enlarged (relative to the baseline sizes) so that the user can manipulate the controls with a less precise touch.

In various embodiments, the modified user interface can include a modification to a physical control. For example, media players may have a dial that adjusts the volume of an audio presentation. The dial may be modified to change a relationship between rotation of the dial and volume change. For example, prior to modification, a ninety degree turn of a volume knob may result in the volume of an audio presentation being doubled. After modification, a one-hundred and eighty degree turn of the volume knob may result in the volume of the audio presentation being doubled. Such a modification may be advantageous in circumstances where a user's fine motor control may be impaired. For example, a user may not be able to finely adjust the volume control knob while running or performing other exercise. Thus, increasing the amount of rotation of the knob to affect a particular change in volume can effectively provide fine motor-like control of the volume knob to the user. Buttons of the user interface can also be modified. For example, prior to modification, pressing a button for any length of time may result in a control action being taken. For example, a physical button (or a region of a touch screen display indicated by a graphical user interface or icon) could be briefly pressed and then released to skip to the next song. After modification, the button may need to be pressed for at least a half second to skip to the next song, thereby preventing inadvertent touches of the button triggering unintentional song skipping. For example, if the user is running, his finger may inadvertently brush a touch screen display with a graphical user interface. If the graphical user interface has been modified as described above, then such a brushing touch should not trigger a skip to the next song (or another control command).

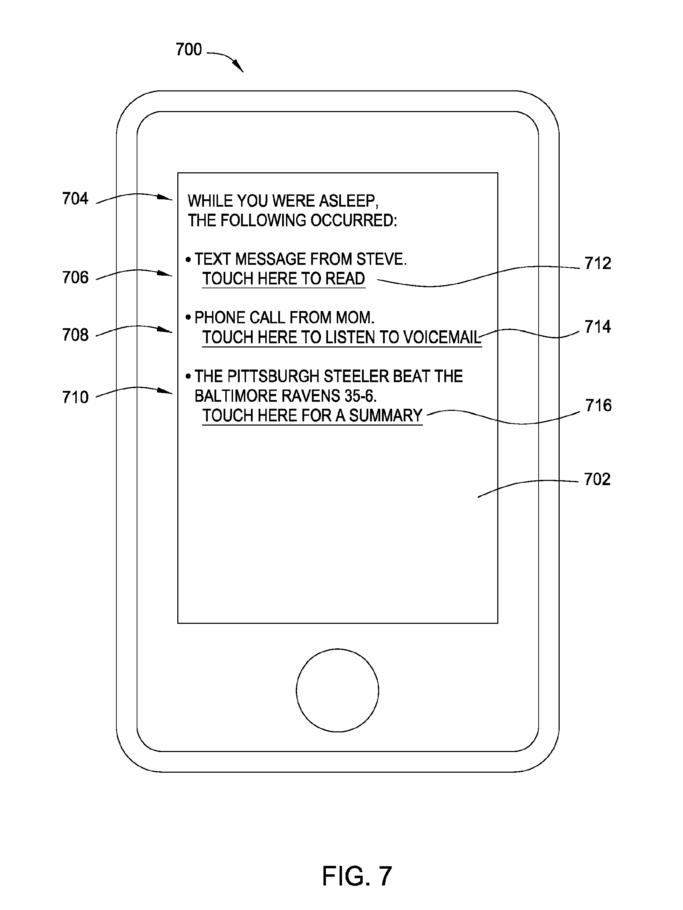

Figure 7 illustrates a smart phone 700 with an embodiment of a controller therein. In the example in which the user is sleeping (as detected by the physiological sensors) the smart phone 700 may modify output audio signals by muting or stopping any audio that is playing when the user falls asleep. For example, if the user is listening to music on the smart phone 700 when he falls asleep, the smart phone 700 could automatically stop playback of the music. In various instances, the smart phone 700 may gradually reduce the volume of the music before stopping the music to avoid an abrupt transition that may awaken the user. The smart phone 700 may also modify audio output signals by muting any ring tones associated with telephone calls, text messages, or other alerts (e.g., alerts from applications running on the smart phone 700) while the user is asleep. The smart phone 700 may also modify video output signals by stopping any video that is playing when the user falls asleep and/or by dimming or de-powering the display screen 702 when the user falls asleep. For example, if the user is watching a video on the display screen 702 when he falls asleep, the smart phone 700 can stop the video and/or dim or turn off the display screen. The smart phone 700 may gradually dim the display screen 702 to avoid abrupt transitions that may awaken the user.

In the event the user receives phone calls, text messages, and/or alerts while asleep, the smart phone 700 can mute or cancel and ringtone or vibration associated with such events and can compile a log of such events and present them to the user when he wakes up. The display screen 702 shown in Figure 7 illustrates an example of an event log. The event log includes a first text field 704 that informs the user that the following summarized events occurred while he was asleep. For example, a second field 706 indicates that a text message was received from Steve. As another example, a third field 708 indicates that a telephone call was received from the user's mother. As another example, a fourth field 710 provides a score for a game that finished while the user was asleep. Additional fields could indicate major news events that occurred while the user was asleep, for example. The event log can include links (e.g., hypertext links) that enable the user to directly access more detailed information related to each event in the event log. For example, the user may touch a first link 712 to go to the text message from Steve. As another example, the user may touch a second link 714 to listen to a voicemail message left by his mother. As yet another example, the user may touch a third link 716 to retrieve additional information about the game.

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, embodiments described as less desirable than other embodiments or prior art implementations with respect to one or more characteristics are not outside the scope of the disclosure and can be desirable for particular applications.

While exemplary embodiments are described above, it is not intended that these embodiments describe all possible forms of the invention. Rather, the words used in the specification are words of description rather than limitation, and it is understood that various changes may be made without departing from the spirit and scope of the invention. Additionally, the features of various implementing embodiments may be combined to form further embodiments of the invention.

In the preceding paragraphs, reference is made to embodiments presented in this disclosure. However, the scope of the present disclosure is not limited to specific described embodiments. Instead, any combination of the preceding features and elements, whether related to different embodiments or not, is contemplated to implement and practice contemplated embodiments. Furthermore, although embodiments disclosed herein may achieve advantages over other possible solutions or over the prior art, whether or not a particular advantage is achieved by a given embodiment is not limiting of the scope of the present disclosure. Thus, the preceding aspects, features, embodiments and advantages are merely illustrative and are not considered elements or limitations of the appended claims except where explicitly recited in a claim(s). Likewise, reference to "the invention" shall not be construed as a generalization of any inventive subject matter disclosed herein and shall not be considered to be an element or limitation of the appended claims except where explicitly recited in a claim(s).

Aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system."

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals *per se,* such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions. The descriptions of the various embodiments of the present invention have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

Embodiments of the invention may be provided to end users through a cloud computing infrastructure. Cloud computing generally refers to the provision of scalable computing resources as a service over a network. More formally, cloud computing may be defined as a computing capability that provides an abstraction between the computing resource and its underlying technical architecture (e.g., servers, storage, networks), enabling convenient, on-demand network access to a shared pool of configurable computing resources that can be rapidly provisioned and released with minimal management effort or service provider interaction. Thus, cloud computing allows a user to access virtual computing resources (e.g., storage, data, applications, and even complete virtualized computing systems) in "the cloud," without regard for the underlying physical systems (or locations of those systems) used to provide the computing resources.

Typically, cloud computing resources are provided to a user on a pay-per-use basis, where users are charged only for the computing resources actually used (e.g. an amount of storage space consumed by a user or a number of virtualized systems instantiated by the user). A user can access any of the resources that reside in the cloud at any time, and from anywhere across the Internet. In context of the present invention, a user may access controller applications (e.g., controller applications that can modify at least one of an audio presentation, a video presentation, and a user interface) or related data available in the cloud. For example, the controller application could execute on a computing system in the cloud and modify at least one of an audio presentation, a video presentation, and a user interface on a media device, such as an iPhone, iPod, smart phone, or docking station. In such a case, the controller application could receive physiological data from physiological sensors being worn by a user and determine a physiological state or change in physiological state at a storage location in the cloud. The controller application can then send to a media device a command signal that can cause the media device to change at least one of an audio presentation, a video presentation, and a user interface, based on the determined physiological state or change in physiological state. Doing so allows a user to access this information from any computing system attached to a network connected to the cloud (e.g., the Internet).

While the foregoing is directed to embodiments of the present invention, other and further embodiments of the invention may be devised without departing from the basic scope thereof, and the scope thereof is determined by the claims that follow.

## Claims

1. A system, comprising:
a computer processor (302);
at least one physiological sensor (114-118, 124-128) in communication with the computer processor, wherein the at least one physiological sensor detects at least one physiological parameter of a user (112, 122);
at least one of a video display adapted to display video images and an audio transducer (104) adapted to generate audio signals; and
a user interface in communication with the computer processor, wherein the user interface includes at least one control that enables the user to control one or more aspects of at least one of video images displayed on the video display and audio signals output by the audio transducer (104),
wherein the computer processor (302) modifies a presentation aspect of at least one of the video images displayed on the video display, a presentation aspect of audio signals output by the audio transducer, and a control on the user interface in response to a change in the detected at least one physiological parameter.

2. The system of claim 1, wherein the presentation aspect of at least one of the video images displayed on the video display comprises a brightness of the video, and wherein the presentation aspect of the at least one audio signal comprises a volume of the audio output by the audio transducer.

3. The system of claim 1 or 2, wherein the at least one physiological sensor comprises at least one of: a heart beat sensor; a thermometer reading a body temperature; and an electroencephalography (EEG) sensor.

4. The system of any of the preceding claims, further comprising a computer-readable storage medium that stores at least one of digital videos for display on the video display and digial audio for output by the audio transducer.

5. The system of any of the preceding claims, further comprising a signal input configured to receive at least one of a video image signal and an audio signal.

6. The system of any of the preceding claims, wherein the user interface comprises a touch screen display (204), wherein the processor outputs for display on the touch screen display a graphical user interface that controls aspects of at least one of the displayed video image and output audio signal, and wherein the processor modifies at least one presentation aspect of the graphical user interface in response to a change in the detected at least one physiological parameter.

7. The system of any of the preceding claims, further comprising a docking port configured to communicate with a portable media player, wherein the computer processor can send control commands to the portable media player through the docking port.

8. A controller, comprising:
a first signal input (300) configured to receive a first physiological signal that indicates a physiological state of a user (112, 122);
a second signal input (304, 306) configured to receive at least one of a video image signal and an audio signal;
a first signal output (312, 314) configured to output a modification control signal; and
computer logic programmed to:
provide to the first signal output the at least one of the video image signal and the audio signal; and
upon the first signal input indicating a change in the physiological state of the user, issue the modification control signal from the first signal output to modify at least one presentation aspect of at least one of a video image signal and an audio signal.

9. The controller of claim 8, further comprising a second output configured to output a user interface signal that provides a user interface display, and wherein the computer logic is further programmed to modify at least one aspect of the user interface display upon the first signal input indicating a change in the physiological state of the user.

10. The controller of claim 9, wherein the change in the physiological state of the user comprises an increased activity level, and wherein the computer logic is programmed to modify the user interface by increasing a size of at least one graphical icon displayed on the user interface display.

11. The controller of any of claims 8 to 10, wherein the second signal input comprises a docking port configured to communicate with a portable media player (108), wherein the portable media player can output the at least one of a video image signal and the audio signal to the second signal input (304, 306).

12. The controller of any of claims 8 to 11, wherein the change in physiological state of the user comprises the user falling asleep, and wherein the computer logic is programmed to stop the at least one of the video image signal and audio signal.

13. The controller of any of claims 8 to 12, wherein the change in physiological state of the user comprises the user increasing a level of physical exertion, and wherein the computer logic is programmed to increase the volume of the audio signal output by the first output.

14. A non-transitory computer readable medium containing a program which, when executed by one or more processors, performs operations comprising:
outputting at least one of a video signal and an audio signal;
receiving at least one indication of a physiological state of a user; and
modifying the output of at least one of the video signal and audio signal upon receiving at least one indication of a change to the physiological state of the user.

15. The non-transitory computer readable medium of claim 14, further comprising:
outputting a graphical user interface to receive at least one user-input control indication; and
modifying the graphical user interface upon receiving the at least one indication of the changed physiological state of the user.
